(19) Europäisches Patentamt
European Patent Office
Office européen des brevets

(11) Numéro de publication: **0 123 668**
**B1**

(12) # FASCICULE DE BREVET EUROPÉEN

(45) Date de publication du fascicule du brevet: **15.10.86**

(51) Int. Cl.⁴: **A 61 K 9/20,** A 61 K 31/47

(21) Numéro de dépôt: **84870054.8**

(22) Date de dépôt: **12.04.84**

(54) **Composition pharmaceutique de glafénine.**

(30) Priorité: **13.04.83 FR 8306004**

(43) Date de publication de la demande: **31.10.84 Bulletin 84/44**

(45) Mention de la délivrance du brevet: **15.10.86 Bulletin 86/42**

(84) Etats contractants désignés: **BE CH DE FR GB IT LI LU NL SE**

(56) Documents cité:
**BE-A-829 197**

**DICTIONNAIRE VIDAL 1982, page 551;**
**PATENTS ABSTRACTS OF JAPAN, vol. 6, no. 202(C-129)(1080), 13 octobre 1982;**
**CHEMICAL ABSTRACTS, vol. 87, 1977, page 386, no. 90734r, Columbus, Ohio, USA.**

(73) Titulaire: **N.V. SOPAR S.A., rue de Thyle, 3a, B-6328 Sart- Dames- Avelines (BE)**

(72) Inventeur: **Vranckx, Henri Armand Léon, Rue Roosendael 3 Boîte 17, B-1190 Forest (BE)**

(74) Mandataire: **Plucker, Guy, OFFICE KIRKPATRICK 4 Square de Meeûs, B-1040 Bruxelles (BE)**



Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne de nouvelles compositions pharmaceutiques et plus particulièrement de nouvelles compositions contenant de l'α-monoglycéride de 4-(2'-carboxyphénylamino)-7-chloroquinoléine de formule:

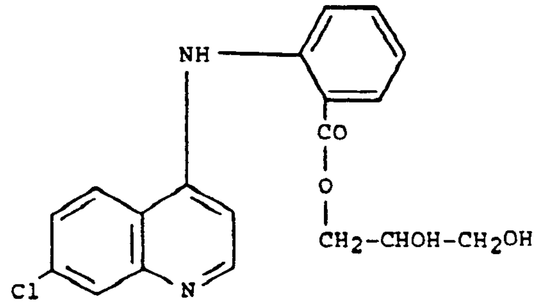

appelé glafénine.

La glafénine est un agent antalgique puissant bien connu qui est commercialisé depuis une vingtaine d'années. Le spectre d'activité de la glafénine lui confère un grand intérêt comme antalgique de grande diffusion, d'où découle la préférence qui est attribuée à une préparation à usage oral.

Toutefois, les préparations pharmaceutiques actuellement connues et commercialisées ont pour inconvénient d'agir assez lentement, parfois avec une insuffisance inattendue. Il a été établi que certains patients (plus particulièrement ceux manifestant de l'achlorhydrie) sont réfractaires à l'assimilation du principe actif des préparations à base de glafénine actuellement connues, la biodisponibilité restant négligeable dans le temps.

On sait que la glafénine se dissout en milieu acide, mais avec les préparations actuellement connues la vitesse de dissolution reste faible. Par ailleurs, la glafénine se dissout aussi en milieu alcalin, mais dans un domaine de pH qui n'entre pas en ligne de compte pour une application thérapeutique.

Il est évidemment souhaitable que la glafénine se dissolve très rapidement après ingestion du médicament de manière à permettre une résorption rapide et, de ce fait, une activité antalgique également rapide. Mais, en outre, une dissolution rapide de la glafénine est également nécessaire pour assurer une résorption globale la plus élevée possible puisque seule la glafénine dissoute peut être résorbée; cette solubilisation n'est possible que pendant le temps relativement bref où le médicament séjourne dans l'estomac. En effet, la glafénine n'est pas soluble dans le milieu neutre ou légèrement alcalin du tractus intestinal.

L'invention a dès lors pour objet de procurer une composition pharmaceutique de glafénine à usage oral, sous forme dosée sèche, et en particulier sous forme de comprimés, assurant une résorption rapide et une biodisponibilité élevée, même lorsque la composition est administrée à des patients qui sont réfractaires à l'assimilation du principle actif des préparations à base de glafénine actuellement connues.

Malgré l'absence de progrès constatée pendant une vingtaine d'années dans les formes de présentation de la glafénine, il est possible de bénéficier des avantages de ce principe actif sans en éprouver les inconvénients découlant d'une résorption trop lente et insuffisante, en administrant une composition de glafénine sous forme de comprimés dont la caractéristique essentielle, par rapport aux compositions connues, consiste en la présence simultanée dans la composition, d'un acide solide pharmaceutiquement acceptable et d'un désintégrant à effet rapide.

La présente invention a pour objet une composition pharmaceutique de glafénine présentée sous forme de comprimé pelliculé se prêtant à l'administration par voie orale, comprenant les constituants suivants:

a) de la glafénine,
b) au moins un acide solide pharmaceutiquement acceptable,
c) au moins un liant,
d) au moins un désintégrant,
e) au moins un agent de pelliculage.

La quantité d'acide solide pharmaceutiquement acceptable doit être choisie en proportion de celle de la glafénine. Le rapport pondéral de l'acide à la glafénine dépend de l'acide choisi, mais peut varier entre des limites assez larges et sera normalement compris entre 1:4 et 1:1. Lorsqu'on utilise de l'acide tartrique, ce rapport pondéral sera de préférence de 1:3 à 1:1,5 et le plus avantageusement d'environ 1:2, ce qui mène, pour 200 mg de glafénine, à des quantités d'acide tartrique de 50 à 200 mg, de préférence de 67 à 134 mg et plus avantageusement d'environ 100 mg.

La présentation sous forme de comprimés conduit à se fixer une limite inférieure et une limite supérieure pour la dimension du comprimé et sa teneur en principle actif. Compte tenu du fait que le médicament sera consommé notamment par des patients âgés, la limite inférieure est imposée, pour la

dimension, par la facilité de préhension et, pour la teneur en principe actif, par la possibilité d'administrer une dose suffisante à l'aide d'un seul comprimé. Pour la limite supérieure, la facilité d'ingestion est décisive. Dans ces conditions, un comprimé rond d'environ 300 à 700 mg ayant une épaisseur à peu près du quart à la moitié du diamètre donne satisfaction.

Dès lors, les quantités des différents constituants des comprimés conformes à l'invention se situent utilement dans les intervalles précisés ci-après.

La quantité de glafénine peut s'échelonner de 100 à 300 mg et de préférence de 150 à 250 mg et est le plus avantageusement de 200 mg environ. Après de nombreuses années de prescription de la glafénine, une dose de cet ordre s'est révélée satisfaisante en pratique. Il convient, de noter que les comprimés conformes à l'invention, qui conduisent à une biodisponibilité plus élevée que celle atteinte avec des comprimés de glafénine de type connu, laissent prévoir, à dose égale, un effet plus marqué que celui de ces derniers.

L'acide solide pharmaceutiquement acceptable est avantageusement choisi parmi les acides organiques de poids moléculaire peu élevé et exempts de longs radicaux aliphatiques, tels que l'acide p-toluènesulfonique, l'acide ascorbique, l'acide fumarique, l'acide citrique et l'acide tartrique. Dans la composition pharmaceutique qui sera décrite et commentée plus en détail par la suite, l'acide solide pharmaceutiquement acceptable est l'acide tartrique racémique conforme aux pharmacopées.

Les quantités cumulées des constituants c), d) et e) rapportées à la somme des constituants a) et b) peuvent être de 1:3 à 1:1, de préférence de 1:2 à 1:1, et spécialement d'environ 2:3. Dans la somme c)+d)+e), la part de c) peut être d'à peu près la moitié, le reste étant formé surtout par le terme d).

Le liant (constituant c)) peut être choisi parmi de nombreux liants utilisés en pharmacie, pour la fabrication de comprimés. On peut citer notamment les amidons, les amidons gélatinisés, les gommes (arabique, adragante, etc.), les gélatines (types A et B), les celluloses (farine ou composé microcristallin), les dérivés cellulosiques (méthyl-, éthyl-, hydroxyéthyl-, hydroxypropyl-, carboxyméthylcellulose), la polyvinylpyrrolidone et ses copolymères (par exemple avec l'acétate de vinyle), les polyéthylèneglycols, les esters de glycérol (comme le palmitostéarate de glycérol). Suivant une forme de réalisation avantageuse de l'invention, le constituant c) est un système formé par un liant synthétique et un liant d'origine naturelle. Le liant synthétique est avantageusement la povidone qui est un homopolymère de la 1-vinyl-2-pyrrolidone. Le liant d'origine naturelle est avantageusement un liant de type cellulosique et plus spécialement la cellulose microcristalline qui associe à sa fonction de liant pour comprimés, un effet désintégrant.

Le constituant d) peut consister en un ou plusieurs agents désintégrants parmi lesquels on peut citer: la crospovidone (polyvinylpolypyrrolidone), les amidons et amidons modifiés (par exemple carboxyméthylamidon), les dérivés cellulosiques, la pectine, la caséïne, l'agar-agar, des silicates complexes (bentonite, montmorillonite, veegum, etc.), l'acide alginique et les alginates.

Il est hautement souhaitable que le désintégrant (constituant d)) soit choisi parmi les désintégrants qui exercent rapidement leur effet. Un agent désintégrant qui s'est révélé spécialement efficace est la crospovidone, qui est un homopolymère réticulé de la 1-vinyl-2-pyrrolidone.

Les comprimés de glafénine de conception traditionnelle sont des comprimés simples, exempts de tout enrobage. Ces comprimés ne présentent aucune saveur désagréable parce que la glafénine dans ces conditions n'est pas soluble dans la cavité buccale.

Par contre, dans les compositions suivant l'invention, la dissolution de la glafénine est très rapide et un comprimé simple, non enrobé, aurait une saveur très déplaisante.

Il importe donc de différer la solubilisation de la glafénine pendant les quelques secondes où le comprimé se trouve en bouche. A cette fin, la présentation sous forme de comprimés pelliculés donne satisfaction. Entre autres agents propres à former cette pellicule, il convient de citer l'hydroxypropyl-méthylcellulose et le macrogol 20.000 (ou polyéthylèneglycol).

La quantité des constituants e) est celle assurant la formation d'une pellicule ayant la durabilité voulue au contact de la salive, mais donnant lieu à une désagrégation rapide dans l'estomac.

Outre les constituants énumérés ci-dessus, la composition suivant l'invention peut comprendre un ou plusieurs agents auxiliaires ou additifs habituellement utilisés dans des compositions de comprimés et en particulier un ou plusieurs lubrifiants et surfactifs.

La fonction de lubrifiant pour le façonnage des comprimés à la presse est assurée avantageusement par le talc et celle de surfactif par le laurylsulfate de sodium.

Dans ses formes de réalisation préférées, l'invention a donc pour objet des comprimés pelliculés de glafénine ayant la constitution suivante:

| Constituant | | Quantité |
|---|---|---|
| Glafénine | | 200 mg |
| Acide solide pharmaceutiquement acceptable | | 50—150 mg |
| Cellulose microcristalline | | 100 mg |
| Crospovidone (polyvinylpolypyrrolidone) | | 82 mg |
| Povidone (polyvinylpyrrolidone) | | 10 mg |
| Talc | | 24 mg |
| Laurylsulfate de sodium pour un noyau-comprimé | | 4 mg |
| Hydroxypropylméthylcellulose | environ | 15 mg |
| Macrogol 20.000 (polyéthylene glycol) pour un comprimé pelliculé | environ | 5 mg |
| | Total | 490—590 mg |

L'acide solide pharmaceutiquement acceptable peut être, dans divers cas particuliers,

— l'acide ascorbique 117 mg
— l'acide fumarique 77 mg
— l'acide citrique 128 mg
— l'acide p-toluènesulfonique 115 mg

Dans sa forme de réalisation particulièrement préférée, l'invention a pour objet un comprimé pelliculé de glafénine, désigné ci-après sous la dénomination EXIDOL®, ayant la constitution suivante:

| Constituant | | Quantité |
|---|---|---|
| Glafénine | | 200 mg |
| Acide tartrique | | 100 mg |
| Cellulose microcristalline | | 100 mg |
| Crospovidone (polyvinylpolypyrrolidone) | | 82 mg |
| Povidone (polyvinylpyrrolidone) | | 10 mg |
| Talc | | 24 mg |
| Laurylsulfate de sodium pour un noyau-comprimé | | 4 mg |
| Hydroxypropylméthylcellulose | environ | 15 mg |
| Macrogol 20.000 (polyéthyléne glycol) pour un comprimé pelliculé | environ | 5 mg |
| | Total | 540 mg |

Ces comprimés pelliculés présentent toutes les propriétés désirées dans les domaines de la vitesse de désintégration, de la cinétique de solubilisation et de la biodisponibilité.

Le Tableau I ci-après fait ressortir que les comprimés pelliculés se délitent rapidement, même après une longue conservation, tant à la température ordinaire qu'à une température correspondant à un climat tropical (40°C). D'autres lots de fabrication ne donnent pas lieu à des valeurs significativement différentes.

TABLEAU 1

| Lot | 81 E 22 | | | | | | |
|---|---|---|---|---|---|---|---|
| Durée de conservation | Départ | 3 mois | | 12 mois | | 18 mois | |
| Température de conservation | — | T. amb. | 40°C | T. amb. | 40°C | T. amb. | 40°C |
| Aspect | C | C | C | C | C | C | N.E. |
| Temps de désintégration | C | C | C | C 34 sec | C 35 sec | C 30 sec | N.E. |
| Dosage de la glafénine | 99,2% | 100,6% | 99,9% | 98,9% | 100,6% | 98,3% | N.E. |
| Recherche de produit de dégradation | — | — | — | néant | néant | néant | N.E. |

C=conforme.
N.E.=non essayé.

Il ressort aussi du Tableau I que la dégradation du principe actif est négligeable puisque la concentration en produits de dégradation reste inférieure à la limite de sensibilité (0,1%).

Dans l'ensemble, les comprimés pelliculés de l'invention ont donc une stabilité à la conservation au moins égale à celle d'autres formes dosées analogues.

Ces temps de désagrégation ont été déterminés suivant les directives de la Pharmacopée Européenne.

La cinétique de solubilisation de la glafénine a fait l'objet de mesures dans différentes conditions expérimentales:

a) à pH 1,2, en simulation du milieu de l'estomac normal,
b) à pH 3, en simulation du milieu de l'estomac insuffisamment acide,

pour quatre formes de préparation de la glafénine

— comprimés conformes à l'invention, sous la dénomination EXIDOL®,
— comprimés suivant la technique antérieure, sous la dénomination GLAFENINE O (comprimés du commerce contenant 200 mg de glafénine et, comme excipients: lacose, amidons, talc, stéarate de magnésium),
— comprimés analogues aux comprimés EXIDOL®, mais sans acide tartrique, ou GLAFENINE I,
— comprimés analogues aux comprimés EXIDOL®, mais sans crospovidone, contenant seulement de l'amidon de maïs comme désintégrant, ou GLAFENINE II.

Les résultats ainsi obtenus constituent les Tableaux II et III et sont repris dans les Fig. 1 et 2 qui portent le pourcentage de solubilisation en ordonnées et le temps en minutes en abscisses et dans lesquelles les symboles sont les suivants:

□ EXIDOL®

■ GLAFENINE O

▲ GLAFENINE I

△ GLAFENINE II

La cinétique de solubilisation de la glafénine a été déterminée suivant le mode opératoire de la pharmacopée américaine (USP XX) à 37°C, dans de l'eau acidulée agitée par une pale tournant à 60 tours par minute, la teneur en glafénine dissoute étant observée par spectrophotométrie d'absorption à 410 nm.

On a également mesuré le temps de désagrégation, le pourcentage de glafénine solubilisée après 3 minutes à pH 1,2, et le pourcentage de glafénine solubilisée après 3 minutes à pH 3 pour les comprimés suivants:

— EXIDOL® (contenant donc 100 mg d'acide tartrique et 82 mg de crospovidone).
— Trois types de comprimés analogues à EXIDOL®, mais dans lesquels les 100 mg d'acide tartrique sont remplacés par

128 mg d'acide citrique,
ou 77 mg d'acide fumarique,
ou 117 mg d'acide ascorbique.

— Deux types de comprimés analogues à EXIDOL® mais dans lesquels les 82 mg de crospovidone sont remplacés par

82 mg de carboxyméthylcellulose sodique réticulée
82 mg de carboxyméthylamidon sodique.

Les résultats ainsi obtenus sont repris aux Tableaux IV et V.

TABLEAU II
Cinétique de solubilisation de la GLAFENINE
pH=1,2

| Temps (minutes) | Pourcentage de solubilisation — Moyenne (écart type) | | | |
|---|---|---|---|---|
| | EXIDOL® | GLAFENINE O | GLAFENINE I | GLAFENINE II |
| 3 | 65,25(7,19) | 28,75(6,09) | 69,75(5,24) | 7,25(0,29) |
| 6 | 79,38(4,19) | 49,88(7,96) | 89,38(3,73) | 12,63(0,25) |
| 9 | 84,25(3,33) | 63,63(9,20) | 93,63(3,66) | 16,88(0,75) |
| 12 | 88,00(2,74) | 74,75(8,53) | 95,38(3,42) | 21,00(0,71) |
| 15 | 91,00(2,04) | 81,50(7,78) | 96,38(3,09) | 25,25(0,87) |
| 18 | 93,13(1,60) | 86,25(6,89) | 96,75(3,10) | 29,75(0,96) |
| 21 | 95,00(1,41) | 89,63(5,94) | 97,25(2,78) | 34,25(1,66) |
| 24 | 96.38(1,38) | 91,88(5,30) | | |
| 27 | 97.88(1,11) | 93,00(4,10) | | |
| 30 | 98,38(1,32) | 94,13(3,71) | | |

TABLEAU III
Cinétique de solubilisation de la GLAFENINE
pH=3

| Temps (minutes) | Pourcentage de solubilisation — Moyenne (écart type) | | | |
|---|---|---|---|---|
| | EXIDOL® | GLAFENINE O | GLAFENINE I | GLAFENINE II |
| 3 | 40,50(1,41) | 1,25(0,29) | 20,75(1,32) | 1,75(2,02) |
| 6 | 48,63(1,03) | 2,38(0,63) | 29,13(3,12) | 5,00(0,41) |
| 9 | 53,13(0,95) | 3,38(0,25) | 32,38(3,57) | 6,03(0,45) |
| 12 | 56,38(0,85) | 4,25(0,65) | 34,13(3,57) | 6,75(0,50) |
| 15 | 58,63(0,85) | 5,13(0,63) | 35,88(3,94) | 7,63(0,48) |
| 18 | 60,38(0,95) | 6,00(0,41) | 37,63(4,21) | 8,75(0,65) |
| 21 | 62,25(1,26) | 6,75(0,54) | 38,75(4,41) | 10,13(0,75) |
| 24 | 63,23(1,11) | 7,38(0,48) | 40,00(4,67) | 11,13(0,75) |
| 27 | 65,00(1,00) | 8,25(0,50) | 40,75(5,07) | 12,63(0,75) |
| 30 | | | 41,75(5,07) | 14,00(1,08) |

TABLEAU IV

| Acide solide pharmaceutiquement acceptable | Temps de désagrégation | Pourcentage de glafénine solubilisée après 3 minutes pH 1,2 | pH 3 |
|---|---|---|---|
| Acide tartrique 100 mg (EXIDOL®) | 30 sec | 65,3 | 40,5 |
| Acide citrique anhydre 128 mg | 20 sec | 71,— | 45,2 |
| Acide fumarique 77 mg | 20 sec | 71,7 | 41,5 |
| Acide ascorbique 117 mg | 35 sec | 71,6 | 35,2 |

TABLEAU V

| Agent désintégrant | Temps de désagrégation | Pourcentage de glafénine solubilisée après 3 minutes pH 1,2 | pH 3 |
|---|---|---|---|
| Crospovidone 82 mg (EXIDOL®) | 30 sec | 65,3 | 40,5 |
| Carboxyméthylcellulose sodique réticulée 82 mg | 2 min 15 sec | 48,7 | 16,7 |
| Carboxyméthylation sodique 82 mg | 2 min 59 sec | 56,8 | 23,3 |

La biodisponibilité a été déterminée de même pour les comprimés EXIDOL® et des comprimés GLAFENINE O.

1. Mode opératoire.

A. Sélection des volontaires

Douze sujets volontaires, dont 4 de sexe féminin, ont participé à l'étude. Ils ont été sélectionnés après un examen médical et un test urinaire comprenant la recherche de glucose, de sang, des protéines, des corps cétoniques, ainsi que la détermination du pH. Tous les sujets retenus sont en bonne santé. Les sujets ne prennent aucun médicament sauf le sujet féminin JM (Diane®). Leurs caractères biométriques sont les suivants (moyenne+extrêmes):

— âge =24 ans (22—28)
— poids=69 kg (47,5—88)
— taille=176 cm (155—187).

Le jour de l'expérience, les volontaires se présentent à jeûn depuis la veille à 20 h. Un cathéter intraveineux est placé dans l'avant-bras (Abbocath 18G) à partir duquel tous les prélèvements sont effectués. L'ingestion de la glafénine est considérée comme temps zéro à partir duquel tous les prélèvements sanguins et les récoltes d'urines sont effectués (Tableau VI). Les échantillons sanguins sont immédiatement centrifugés après leur prélèvement et les plasmas congelés. Les volumes urinaires de chaque fraction ont été immédiatement mesurés et une aliquote a été prélevée en vue des dosages ultérieurs. Durant les huits heures d'expérimentation, les volontaires sont sous surveillance médicale. Durant ce temps, un apport liquidien constitué d'eau "Spa Reine®" et une alimentation standardisée ont été administrés selon le protocole présenté au Tableau VI. La tolérance a été excellente au cours des expérimentations pour tous les volontaires.

B. Formes médicamenteuses administrées

Les deux formes orales de glafénine sont les suivantes:

1. GLAFENINE O, administrée en l'occurrence sous forme de Gilfanan® Roussel (représentatif des produits du commerce); glafénine 200 mg par comprimé,

excipient: lactose, amidons, talc, stéarate de magnésium,

lot: 80C31 D pour 8 volontaires
81A09 pour volontaires BM, GP, CA, SD.

2. EXIDOL®; glafénine 200 mg par comprimé,

lot: 81A22.

Les deux formes orales de glafénine ont été administrées aux volontaires groupés par trois, à la dose de *400 mg* (deux comprimés), selon la séquence GLAFENINE O; EXIDOL® à l'intervalle d'une semaine pour le premier et le troisième groupe et selon la séquence EXIDOL®, GLAFENINE O pour le second et le quatrième groupe, toujours à l'intervalle d'une semaine.

C. Dosage de l'acide glafénique

La glafénine administrée par voie orale est rapidement absorbée, une fois solubilisée en milieu acide. Dès sa résorption, la glafénine est rapidement métabolisée en acide glafénique qui est la forme physiologiquement utile du principe actif, avec disparition complète et rapide de la glafénine dans le plasma.

Etant donné cette métabolisation intense et rapide, le dosage plasmatique et urinaire de l'acide glafénique plutôt que celui de la glafénine s'impose. Le dosage a donc été effectué selon la méthode proposée par F. Moolenaar et al., (Int. J. Pharm. *4* (1980) 195—230) en utilisant la chromatographie liquide à haute performance (HPLC). Les conditions opératoires sont les suivantes:

— Pompe Pye Unicam LC-XPD, débit 1 ml/min.
— Détecteur UV Pye Unicam, 365 nm (sensibilité de 0,01 à 0,16 suivant les concentrations).
— Colonne Macherey-Nagel Polygosil 7,5 C18 25 cm.
— Précolonne 5 cm Vydac RP 18.
— Phase mobile: Tampon Phosphate 0,007 M pH 6,6 40.
Méthanol 60.
— Vanne d'injection Rhéodyn—7120 (volume de boucle 100 microlitres).

2. Résultats et conclusions

Les taux plasmatiques d'acide glafénique obtenus suite à l'administration de deux comprimés de GLAFENINE O ou d'EXIDOL® à 12 volontaires sont repris dans les Tableaux VII et VIII. Les mêmes taux plasmatiques ont été portés en graphique et sont repris aux Fig. 3 et 4 portant en ordonnées la concentration plasmatique en acide glafénique en µg par ml et en abscisses le temps en minutes, la courbe A étant relative à GLAFENINE O et la courbe B à EXIDOL®. La Fig. 3 concerne les résultats moyens pour les 12 sujets et la Fig. 4 se rapporte aux résultats pour le sujet SD manifestant de l'achlorhydrie.

1. A partir de ces résultats, le calcul de l'aire sous la courbe comprise entre le temps zéro et 480 min, a été effectué par la méthode des trapèzes pour chaque forme orale de glafénine. Les résultats obtenus sont repris dans le Tableau IX. La biodisponibilité relative (Fr) de la glafénine d'EXIDOL® par rapport à la glafénine de référence (GLAFENINE O) a été calculée par le rapport des aires sous les courbes de la forme testée par rapport au produit de référence. Ces résultats sont également repris dans le Tableau IX. A partir de ces résultats, on peut observer que la glafénine d'EXIDOL® présente une biodisponibilité relative supérieure de 50% à celle de GLAFENINE O. Notons cependant que si l'on excepte le volontaire SD (dont la biodisponibilité relative est nettement supérieure à celles des autres volontaires), la glafénine d'EXIDOL® présente encore une biodisponibilité relative supérieure de 25% à celle de GLAFENINE O.

2. Les pics de concentration plasmatique maximale (tmax) apparaissent pour GLAFENINE O ainsi que pour EXIDOL® aux temps 40 min, 60 min et 80 min. Dans les conditions testées, EXIDOL® ne serait donc pas différente de la forme de référence quant à la vitesse d'apparition de "tmax".

3. Les concentrations plasmatiques maximales (Cp max) en acide glafénique varient pour EXIDOL® entre 8,61 et 34,31 µg/ml et pour GLAFENINE O entre 3,10* et 26,05 µg/ml, les concentrations étant légèrement plus élevées pour le produit de l'invention que pour celui conforme à la technique antérieure.

3. Effets secondaires

Aucun effet secondaire tant objectif que subjectif n'a été noté durant cette étude.

* 8,18 µg/ml si l'on exclut le volontaire SD dont la résorption de GLAFENINE O s'avère particulièrement faible.

TABLEAU VI

Protocole expérimental

Glafénine 400 mg

| Temps | Sang | Urines | Aliments | Boissons (Spa Reine®) |
|---|---|---|---|---|
| To (+20 ml) | +(1) | +(1) | | 250 ml |
| To + 10 min | +(2) | | | |
| To + 20 min | +(3) | | | |
| To + 40 min | +(4) | | | |
| To + 60 min | +(5) | +(2) | déjeuner | 250 ml |
| To + 20 min | +(6) | | | |
| To + 100 min | +(7) | +(3) | | 250 ml |
| To + 120 min | +(8) | | | 250 ml |
| To +150 min | +(9) | +(4) | dîner | 250 ml |
| To + 3 h | +(10) | +(5) | | 250 ml |
| To + 4 h | +(11) | | | 250 ml |
| To + 6 h | +(12) | +(6) | | |
| To + 8 h | +(13) | +(7) | | |
| To + 24 h | | +(8) | | |

TABLEAU VII

Concentrations plasmatiques d'acide glafénique exprimées en µg/ml suite à la prise orale de 400 mg de glafénine d'EXIDOL® par douze volontaires

| Tps(h) (min) | 0,167 10 | 0,33 20 | 0,67 40 | 1 60 | 1,33 80 | 1,67 100 | 2 120 | 2,5 150 | 3 180 | 4 240 | 6 360 | 8 480 |
|---|---|---|---|---|---|---|---|---|---|---|---|---|
| HF | 0,67 | 4,73 | 22,68 | 34,31 | 22,20 | 6,15 | 11,90 | 3,86 | 2,02 | 1,35 | 0,484 | 0,356 |
| BJL | 0,26 | 5,80 | 15,71 | 17,38 | 14,75 | 8,96 | 5,09 | 2,63 | 2,43 | 1,38 | 0,71 | 0,45 |
| LP | 2,07 | 7,42 | 15,69 | 14,90 | 11,58 | 5,15 | 2,46 | 1,48 | 1,26 | 0,72 | 0,47 | 0,31 |
| BF | 0,81 | 4,19 | 9,96 | 16,25 | 18,14 | 10,26 | 4,62 | 1,99 | 1,57 | 1,02 | 0,51 | 0,30 |
| SP | 0,51 | 3,62 | 6,49 | 7,21 | 8,61 | 7,01 | 3,47 | 1,97 | 1,50 | 1,58 | 0,64 | 0,36 |
| VHC | 0,95 | 5,38 | 13,43 | 14,96 | 11,55 | 7,01 | 4,15 | 2,37 | 1,58 | 1,20 | 0,82 | 0,48 |
| JM | 0,36 | 4,20 | 8,83 | 6,78 | 9,80 | 4,84 | 3,75 | 1,79 | 1,09 | 0,73 | 0,39 | 0,18 |
| EN | 0,67 | 3,93 | 9,67 | 11,37 | 7,33 | 4,13 | 2,90 | 1,79 | 1,08 | 0,78 | 0,56 | 0,23 |
| CA | 1,58 | 6,90 | 14,58 | 12,33 | 7,17 | 3,53 | 2,82 | 1,75 | 1,51 | 1,00 | 0,53 | 0,46 |
| GP | 0,79 | 6,03 | 13,81 | 12,01 | 9,59 | 4,30 | 3,09 | 1,37 | 1,15 | 0,72 | 0,39 | 0,25 |
| SD | 2,53 | 9,65 | 16,09 | 13,94 | 11,86 | 5,90 | 3,49 | 2,28 | 1,58 | 1,04 | 0,34 | 0,16 |
| BM | 2,52 | — | 18,53 | 29,05 | 18,09 | 7,56 | 4,04 | 2,52 | 1,88 | 1,61 | 0,78 | 0,52 |

TABLEAU VIII

Concentrations plasmatiques d'acide glafénique exprimées en µg/ml suite à la prise orale de 400 mg de glafénine de GLAFENINE O par douze volontaires

| Tps(h) (min) | 0,167 10 | 0,33 20 | 0,67 40 | 1 60 | 1,33 80 | 1,67 100 | 2 120 | 2,5 150 | 3 180 | 4 240 | 6 360 | 8 480 |
|---|---|---|---|---|---|---|---|---|---|---|---|---|
| HF | 0,90 | 3,10 | 13,21 | 26,05 | 22,29 | 8,25 | 4,52 | 2,28 | 1,77 | 1,20 | 0,61 | 0,41 |
| BJL | 0,00 | 0,13 | 4,48 | 8,23 | 11,34 | 6,21 | 3,61 | 2,37 | 1,45 | 1,08 | 0,59 | 0,29 |
| LP | 0,58 | 2,37 | 5,14 | 8,91 | 11,25 | 6,62 | 4,30 | 2,41 | 1,66 | 0,95 | 0,51 | 0,34 |
| BF | 0,08 | 0,90 | 9,65 | 11,51 | 7,43 | 3,99 | 1,89 | 1,48 | 0,54 | 0,55 | 0,37 | 0,31 |
| SP | 0,71 | 6,94 | 17,00 | 13,55 | 8,85 | 4,87 | 2,98 | 1,65 | 1,09 | 0,70 | 0,46 | 0,30 |
| VHC | 1,49 | 12,30 | 17,47 | 17,17 | 11,44 | 4,67 | 3,31 | 1,88 | 1,35 | 0,87 | 0,45 | 0,19 |
| JM | 0,51 | 3,81 | 8,17 | 8,08 | 6,90 | 3,24 | 1,98 | 1,12 | 0,79 | 0,67 | 0,27 | 0,17 |
| EN | 0,41 | 7,31 | 15,19 | 11,81 | 8,17 | 5,14 | 3,38 | 2,01 | 1,25 | 0,77 | 0,53 | 0,28 |
| CA | 0,00 | 0,40 | 5,83 | 8,34 | 6,99 | 3,52 | 1,81 | 1,18 | 1,01 | 0,59 | 0,31 | 0,27 |
| GP | 0,09 | 0,92 | 5,84 | 9,38 | 12,84 | 10,29 | 4,53 | 1,98 | 1,16 | 0,72 | 0,47 | 0,24 |
| SD | 0,85 | 2,96 | 3,10 | 1,71 | 1,38 | 1,13 | 0,76 | 0,60 | 0,45 | 0,43 | 0,24 | 0,29 |
| BM | 0,48 | 3,06 | 8,32 | 16,98 | 24,06 | 14,76 | 6,62 | 3,45 | 2,46 | 1,57 | 0,59 | 1,06 |

TABLEAU IX
Aires sous les courbes de concentrations en f(temps) exprimées en µg · h · $ml^{-1}$ suite à l'administration de 400 mg de glafénine à douze volontaires
Fr=coefficient de biodisponibilité relative

| Sujets | EXIDOL® | GLAFENINE (O) | Fr. |
|---|---|---|---|
| HF | 41,39 | 31,88 | 1,30 |
| BJL | 29,61 | 16,99 | 1,74 |
| LP | 22,85 | 18,31 | 1,25 |
| BF | 26,27 | 14,89 | 1,76 |
| SP | 18,30 | 21,72 | 0,84 |
| VHC | 25,10 | 25,85 | 0,97 |
| JM | 16,51 | 13,49 | 1,22 |
| EN | 17,30 | 21,16 | 0,82 |
| CA | 20,88 | 12,20 | 1,71 |
| GP | 19,76 | 19,40 | 1,02 |
| SD | 24,75 | 5,63 | 4,40 |
| BM | 35,44 | 33,12 | 1,07 |
| $X \pm s_x$ (N=12) | 24,85±7,54 | 19,55±7,96 | 1,51±0,97 |
| $X \pm s_x$ (N=11) (exc. vol. SD) | 24,86±7,91 | 20,82±6,97 | 1,25±0,35 |

On trouvera ci-dessous l'interprétation statistique de ces résultats, suivant le test de t pour des observations appariées.

TABLEAU X

1. Comparaison des taux plasmatiques d'acide glafénique

| Temps (min) | t observé | Signification ($t_{11}$, 5%=2,201) |
|---|---|---|
| 10 | 2,513 | S. |
| 20 | 1,515 | N.S. |
| 40 | 1,043 | N.S. |
| 60 | 2,404 | S. |
| 80 | 1,042 | N.S. |
| 100 | 0,168 | N.S. |
| 120 | 1,321 | N.S. |
| 150 | 1,148 | N.S. |
| 180 | 1,927 | N.S. |
| 240 | 2,831 | S. |
| 360 | 2,544 | S. |
| 480 | −0,132 | N.S. |

S=significatif
NS=non significatif

La statistique ne permet pas de conclure à une différence significative aux temps 20 et 40 minutes en raison des variations importantes des pics plasmatiques.

2. Comparison des surfaces sous la courbe
t observé=2,602 S.

Même si le volontaire SD est exclu de l'essai, le t observé=2,304 est significatif ($t_{10}$, 5%=2,228). L'amélioration de la biodisponibilité se traduit par une obtention plus rapide de taux plasmatiques élevés pendant la première heure après administration.

TABLEAU XI

| Temps (min) | Hauteur du pic dû à EXIDOL® exprimée en % du pic correspondant dû à GLAFENINE O |
|---|---|
| 10 | 224 |
| 20 | 153 |
| 40 | 146 |
| 60 | 134 |
| 80 | 113 |
| 100 | 103 |
| 120 | 131 |
| 150 | 115 |
| 180 | 124 |
| 240 | 130 |
| 360 | 122 |
| 480 | 97 |

Le Cmax de GLAFENINE O, qui se situe à 60 min, est atteint en 35 min par le produit de l'invention (EXIDOL®).

Malgré les avantages d'EXIDOL® sur GLAFENINE O, il restait intéressant d'établir une comparaison avec une composition pharmaceutique de glafénine en suspension.

Les taux sanguins de glafénine (acide glafénique) suite à une administration de glafénine en suspension ont déjà été déterminés par Moolenaar et al., International Journal of Pharmaceutics *4* (1980) 200.

Le Tableau XII permet cette comparaison.

TABLEAU XII

| Concentration plasmatique (µg/ml aux temps (min)) | GLAFENINE O | Suspension aqueuse de glefénine | EXIDOL® |
|---|---|---|---|
| 30 | 6,57* | 9,2 | 9,71* |
| 60 | 11,81 | 12,8 | 15,87 |
| 120 | 3,31 | 4,6 | 4,32 |
| 180 | 1,25 | 1,6 | 1,55 |
| 240 | 0,84 | 1,1 | 1,09 |

* Valeurs interpolées.

Il ressort du Tableau XII qu'EXIDOL® l'emporte en biodisponibilité également sur une suspension aqueuse de glafénine qu'on pourrait à priori envisager comme très active.

L'ensemble des résultats présentés ci'dessus fait ressortir que le comprimé de glafénine conforme à l'invention assure une biodisponibilité plus complète et plus rapide que celle attainte avec les comprimés de conception classique.

Cette supériorité du produit de l'invention est surprenante et ne s'explique pas directement par l'apport de l'acide tartrique (ou d'un autre acide solide pharmaceutiquement acceptable). En effet, il étain déjà connu que la glafénine se dissout mieux en milieu acide, mais l'acide ajouté suivant l'invention est relativement faible et la quantité qui en est prise est à peine supérieure à l'équivalent par rapport à la glafénine (si on considère qu'une seule fonction acide est consommée par une base en fait monovalente) et est petite, à savoir de 2/3 de millimole, en comparaison de la réserve acide de l'estomac.

A cours d'un essai clinique ouvert réalisé sur 959 patients ambulatoires, EXIDOL® s'est montré statistiquement supérieur en rapidité et intensité d'action, ainsi qu'en tolérance, à des préparations du commerce à base de flurbiprofène, glafénine, acide acétylsalicyclique, paracètamol, bromure de diméthyl-N-octyle, caféine et tartrate d'ergotamine, floctafénine propyphénazone. La rapidité de l'action a également été significativement améliorée vis-à-vis de spécialités à base de naproxène, oxyphénbutazone, diclofénacine sodique, kétoprofène, diflunisal, aspirine et phénacétine, butylscopolamine.

En outre, une étude randomisée en double aveugle a été réalisée chez 27 patients en unité de stomatologie. Après avoir subi l'extraction d'une dent de sagesse incluse, ces patients éprouvaient une douleur modérée à sévère lors de l'administration de 2 comprimés, soit EXIDOL®, soit GLAFENINE O. L'intensité de la douleur est évaluée sur une échelle comparative 30 minutes après la prise du médicament. L'efficacité de ce dernier est appréciée par le coefficient PID (Pain Intensity Difference), compris entre 0 et 10, calculé à partir des intensités de douleur au départ et après 30 minutes. Ces résultats sont indiqués au Tableau XIII.

TABLEAU XIII

| EXIDOL® | | GLAFENINE O | |
|---|---|---|---|
| Patient | P.I.D. | Patient | P.I.D. |
| V.A.R. | 6,0 | C.K. | 2 |
| C.K. | 1,8 | C.D. | 0 |
| S.J. | 7 | C.M. | 5,6 |
| V.A. | 3,2 | C.R. | 2,5 |
| B.G. | 4,1 | C.F. | 5,5 |
| V.A. | 1,3 | V.Q.I. | 0 |
| V.M.A. | 4,8 | J.A. | 0 |
| D.C.J. | 8,2 | D.C. | 1,9 |
| T.M. | 0 | C.P. | 7 |
| M.P. | 7,8 | A.G. | 3,1 |
| V.E. | 1,7 | G.F. | 1,3 |
| R.R. | 1,8 | V.E. | 0 |
| B.G. | 9,8 | B.P. | 0 |
| | | P.M. | 2,5 |
| Moyenne | 4,42 | Moyenne | 2,24 |
| Variance | 9,61 | Variance | 5,56 |
| Ecart type sur la moyenne | 0,86 | Ecart type sur la moyenne | 0,63 |

Le test de F de Snedecor pour la comparaison des variances (F=1,73) montre que l'hypothèse d'égalité dans les 2 sous-groupes est vérifiée (p<5%).

Les P.I.D. moyens peuvent donc être valablement comparés. Le test de t de Student sur les P.I.D. moyens (t=2,05) montre que l'hypothèse d'égalité doit être rejetée ($p<5\%$).

Autrement dit, l'intensité de l'action antalgique d'EXIDOL® est statistiquement plus élevée de manière significative que celle de GLAFENINE O une demi-heure après la prise du médicament. Ceci est en parfaite concordance avec les résultats obtenus au cours de l'essai de biodisponibilité.

**Revendications**

1. Composition pharmaceutique de glafénine présentée sous forme de comprimé pelliculé se prêtant à l'administration par voie orale, caractérisée en ce qu'elle comprend les constituants suivants:

a) de la glafénine, [a-monoglycéride de 4-(2'-carboxyphénylamino)-7-chloroquinoline]
b) au moins un acide solide pharmaceutiquement acceptable,
c) au moins un liant,
d) au moins un désintégrant,
e) au moins un agent de pelliculage,

le rapport pondéral du constituant b) au constituant a) étant de 1:4 à 1:1.

2. Composition pharmaceutique suivant la revendication 1, caractérisée en ce que le rapport pondéral du constituant b) au constituant a) est de 1:2.

3. Composition pharmaceutique suivant l'une quelconque des revendications 1 et 2, caractérisée en ce que le rapport pondéral de la somme des constituants c), d) et e) à la somme des constituants a) et b) est de 1:3 à 1:1.

4. Composition pharmaceutique suivant la revendication 3, caractérisée en ce que le rapport pondéral de la somme des constituants c), d) et e) à la somme des constituants a) et b) est de 1:2 à 1:1.

5. Composition pharmaceutique suivant l'une quelconque des revendications précédentes, caractérisée en ce que la quantité de glafénine est de 100 à 300 mg par comprimé.

6. Composition pharmaceutique suivant la revendication 5, caractérisée en ce que la quantité de glafénine est de 200 mg per comprimé.

7. Composition pharmaceutique suivant l'une quelconque des revendications précédentes, caractérisée en ce que le constituant b) est choisi entre l'acide p-toluènesulfonique, l'acide ascorbique, l'acide fumarique, l'acide citrique, l'acide L-tartrique, l'acide D-tartrique et l'acide tartrique racémique.

8. Composition pharmaceutique suivant la revendication 7, caractérisée en ce que le constituant b) est l'acide tartrique racémique.

9. Composition pharmaceutique suivant la revendication 7, caractérisée en ce que le constituant b) est l'acide citrique.

10. Composition pharmaceutique suivant la revendication 7, caractérisée en ce que le constituant b) est l'acide fumarique.

11. Composition pharmaceutique suivant la revendication 7, caractérisée en ce que le constituant b) est l'acide ascorbique.

12. Composition pharmaceutique suivant l'une quelconque des revendications précédentes, caractérisée en ce que le constituant c) est un système formé par un liant synthétique et un liant d'origine naturelle.

13. Composition pharmaceutique suivant la revendication 12, caractérisée en ce que le liant synthétique est la povidone (polyvinylpyrrolidone).

14. Composition pharmaceutique suivant l'une quelconque des revendications 12 et 13, caractérisée en ce que le liant d'origine naturelle est la cellulose microcristalline.

15. Composition pharmaceutique suivant l'une quelconque des revendications précédentes, caractérisée en ce que le constituant d) est la crospovidone (polyvinylpolypyrrolidone).

16. Composition pharmaceutique suivant l'une quelconque des revendications précédentes, caractérisée en ce que le constituant e) est un système formé par de l'hydroxypropylméthylcellulose et du macrogol 20.000 (polyéthylèneglycol).

17. Composition pharmaceutique suivant l'une quelconque des revendications précédentes, caractérisée en ce qu'elle comprend, en outre, au moins un lubrifiant.

18. Composition pharmaceutique suivant la revendication 17, caractérisée en ce que le lubrifiant est le talc.

19. Composition pharmaceutique suivant l'une quelconque des revendications précédentes, caractérisée en ce qu'elle comprend, en outre, au moins un surfactif.

20. Composition pharmaceutique suivant la revendication 19, caractérisée en ce que le surfactif est le laurylsulfate de sodium.

21. Composition pharmaceutique suivant la revendication 1, caractérisée en ce qu'elle comprend:

| Constituant | | Quantité |
|---|---|---|
| Glafénine | | 200 mg |
| Acide tartrique | | 100 mg |
| Cellulose microcristalline | | 100 mg |
| Crospovidone (polyvinylpolypyrrolidone) | | 82 mg |
| Povidone (polyvinylpyrrolidone) | | 10 mg |
| Talc | | 24 mg |
| Laurylsulfate de sodium | | 4 mg |
| pour un noyau-comprimé | | |
| Hydroxypropylméthylcellulose | environ | 15 mg |
| Macrogol 20.000 (polyéthylèneglycol) | environ | 5 mg |
| pour un comprimé pelliculé | | |
| | Total | 540 mg |

**Patentansprüche**

1. Pharmazeutische Zusammensetzung von Glafenin in Form einer Filmtablette, bzw. überzogenen Tablette, die sich zur oralen Verabreichung eignet, dadurch gekennzeichnet, dass sie die folgenden Bestandteile umfasst:

a) Glafenin [4-[(2-carboxyphenyl)amino]-7-chlorochinolin-α-monoglyzerid]
b) wenigstens eine feste, pharmazeutisch annehmbare Säure
c) wenigstens ein Bindemittel
d) wenigstens ein Zerfallmittel
e) wenigstens ein Umhüllungsmittel,

wobei das Gewichtsverhältnis des Bestandteils b) zur Bestandteil a) von 1:4 bis 1:1 beträgt.

2. Pharmazeutische Zusammensetzung gemäss Anspruch 1, dadurch gekennzeichnet, dass das Gewichtsverhältnis des Bestandteils b) zur Bestandteil a) 1:2 ist.

3. Pharmazeutische Zusammensetzung gemäss einem der Ansprüche 1 und 2, dadurch gekennzeichnet, dass das Gewichtsverhältnis der Summe der Bestandteile c), d) und e) zur Summe der Bestandteile a) und b) von 1:3 bis 1:1 ist.

4. Pharmazeutische Zusammensetzung gemäss Anspruch 3, dadurch gekennzeichnet, dass das Gewichtsverhältnis der Summe der Bestandteile c), d) und e) zur Summe der Bestandteile a) und b) von 1:2 bis 1:1 ist.

5. Pharmazeutische Zusammensetzung gemäss einem der vorhergehenden Ansprüche, dadurch gekennzeichnet, dass die Menge an Glafenin 100 bis 300 mg je Tablette begrägt.

6. Pharmazeutische Zusammensetzung gemäss Anspruch 5, dadurch gekennzeichnet, dass die Menge an Glafenin 200 mg je Tablette beträgt.

7. Pharmazeutische Zusammensetzung gemäss einem der vorhergehenden Ansprüche, dadurch gekennzeichnet, dass der Bestandteil b) ausgewäht ist unter der para-Toluolsulfonsäure, der Ascorbinsäure, der Fumarsäure, der Zitronensäure, der L-Weinsäure, der D-Weinsäure und der racemischen Weinsäure.

8. Pharmazeutische Zusammensetzung gemäss Anspruch 7, dadurch gekennzeichnet, dass der Bestandteil b) die racemische Weinsäure ist.

9. Pharmazeutische Zusammensetzung gemäss Anspruch 7, dadurch gekennzeichnet, dass der Bestandteil b) die Zitronensäure ist.

10. Pharmazeutische Zusammensetzung gemäss Anspruch 7, dadurch gekennzeichnet, dass der Bestandteil b) die Fumarsäure ist.

11. Pharmazeutische Zusammensetzung gemäss Anspruch 7, dadurch gekennzeichnet, dass der Bestandteil b) die Ascorbinsäure ist.

12. Pharmazeutische Zusammensetzung gemäss einem der vorhergehenden Ansprüche, dadurch gekennzeichnet, dass der Bestandteil c) ein System ist, gebildet aus einem synthetischen Bindemittel und einem Bindemittel natürlichen Ursprungs.

13. Pharmazeutische Zusammensetzung gemäss Anspruch 12, dadurch gekennzeichnet, dass das synthetische Bindemittel das Povidone (polyvinylpyrrolidon) ist.

14. Pharmazeutische Zusammensetzung gemäss einem der Ansprüche 12 und 13, dadurch gekennzeichnet, dass das Bindemittel natürlichen Ursprungs die mikrokristalline Cellulose ist.

15. Pharmazeutische Zusammensetzung gemäss einem der vorhergehenden Ansprüche, dadurch gekennzeichnet, dass der Bestandteil d) das Crospovidone (polyvinylpolypyrrolidon) ist.

16. Pharmazeutische Zusammensetzung gemäss einem der vorhergehenden Ansprüche, dadurch gekennzeichnet, dass der Bestandteil e) ein system gebildet aus der Hydroxypropylmethylcellulose und Macrogol 20.000 ist.

17. Pharmazeutische Zusammensetzung gemäss einem der vorhergehenden Ansprüche, dadurch gekennzeichnet, dass sie ausserdem mindestems ein Gleitmittel enthält.

18. Pharmazeutische Zusammensetzung gemäss Anspruch 17, dadurch gekennzeichnet, dass das Gleitmittel der Talk ist.

19. Pharmazeutische Zusammensetzung gemäss einem der vorhergehenden Ansprüche, dadurch gekennzeichnet, dass sie ausserdem mindestens ein oberflächenaktives Mittel enthält.

20. Pharmazeutische Zusammensetzung gemäss Anspruch 19, dadurch gekennzeichnet, dass das oberflächenaktives Mittel das Natriumlaurylsulfat ist.

21. Pharmazeutische Zusammensetzung gemäss Anspruch 1, dadurch gekennzeichnet, dass sie umfasst:

| Bestandteil | | Menge |
|---|---|---|
| Glafenin | | 200 mg |
| Weinsäure | | 100 mg |
| Mikrokristalline Cellulose | | 100 mg |
| Crospovidone (polyvinylpolypyrrolidon) | | 82 mg |
| Povidone (polyvinylpyrrolidon) | | 10 mg |
| Talk | | 24 mg |
| Natriumlaurylsulfat für einen Tablettenkern | | 4 mg |
| Hydroxypropylmethylcellulose | ungefähr | 15 mg |
| Macrogol 20.000 (polyethylenglycol) | ungefähr | 5 mg |
| für eine Filmtablette | insgesamt | 540 mg |

## Claims

1. Pharmaceutical composition containing glafenine, which takes the form of a film-coated tablet suitable for oral administration, characterized in that it contains the following constituents:

a) glafenine [4-(2'-carboxyphenylamino)-7-chloroquinoline-α-monoglyceride],
b) at least one pharmaceutically acceptable solid acid,
c) at least one binder,
d) at least one disintegrating agent,
e) at least one film-forming agent,

the weight ratio of the constituent b) to the constituent a) being from 1:4 to 1:1.

2. Pharmaceutical composition according to Claim 1, characterized in that the weight ratio of the constituent b) to the constituent a) is 1:2.

3. Pharmaceutical composition according to either one of Claims 1 and 2, characterized in that the weight ratio of the sum of the constituents c), d) and e) to the sum of the constituents a) and b) is from 1:3 to 1:1.

4. Pharmaceutical composition according to Claim 3, characterized in that the weight ratio of the sum of the constituents c), d) and e), to the sum of the constituents a) and b) is from 1:2 to 1:1.

5. Pharmaceutical composition according to any one of the preceding claims, characterized in that the amount of glafenine is from 100 to 300 mg per tablet.

6. Pharmaceutical composition according to claim 5, characterized in that the amount of glafenine is 200 mg per tablet.

7. Pharmaceutical composition according to any one of the preceding claims, characterized in that the constituent b) is chosen from p-toluenesulphonic acid, ascorbic acid, fumaric acid, citric acid, L-tartaric acid, D-tartaric acid and racemic tartaric acid.

8. Pharmaceutical composition according to Claim 7, characterized in that the constituent b) is racemic tartaric acid.

9. Pharmaceutical composition according to Claim 7, characterized in that the constituent b) is citric acid.

10. Pharmaceutical composition according to Claim 7, characterized in that the constituent b) is fumaric acid.

11. Pharmaceutical composition according to Claim 7, characterized in that the constituent b) is ascorbic acid.

12. Pharmaceutical composition according to any one of the preceding claims, characterized in that the constituent c) is a system consisting of a synthetic binder and a binder of natural origin.

13. Pharmaceutical composition according to Claim 12, characterized in that the synthetic binder is povidone (polyvinylpyrrolidone).

14. Pharmaceutical composition according to either one of Claims 12 and 13, characterized in that the binder of natural origin is microcrystalline cellulose.

15. Pharmaceutical composition according to any one of the preceding claims, characterized in that constituent d) is crospovidone (polyvinylpolypyrrolidone).

16. Pharmaceutical composition according to any one of the preceding claims, characterized in that the constituent e) is a system consisting of hydroxypropylmethylcellulose and macrogol 20,000 (polyethylene glycol).

17. Pharmaceutical composition according to any one of the preceding claims, characterized in that it contains, in addition, at least one lubricant.

18. Pharmaceutical composition according to Claim 17, characterized in that the lubricant is talc.

19. Pharmaceutical composition according to any one of the preceding claims, characterized in that it contains, in addition, at least one surfactant.

20. Pharmaceutical composition according to Claim 19, characterized in that the surfactant is sodium lauryl sulphate.

21. Pharmaceutical composition according to Claim 1, characterized in that it contains:

| Constituent | | Amount |
|---|---|---|
| Glafenine | | 200 mg |
| Tartaric acid | | 100 mg |
| Microcrystalline cellulose | | 100 mg |
| Crospovidone (polyvinylpolypyrrolidone) | | 82 mg |
| Povidone (polyvinylpyrrolidone) | | 10 mg |
| Talc | | 24 mg |
| Sodium lauryl sulphate for a core tablet | | 4 mg |
| Hydroxypropylmethyl cellulose | approx. | 15 mg |
| Macrogol 20,000 (polyethylene glycol) for a film-coated tablet | approx. | 5 mg |
| | Total | 540 mg |

Fig. 1.

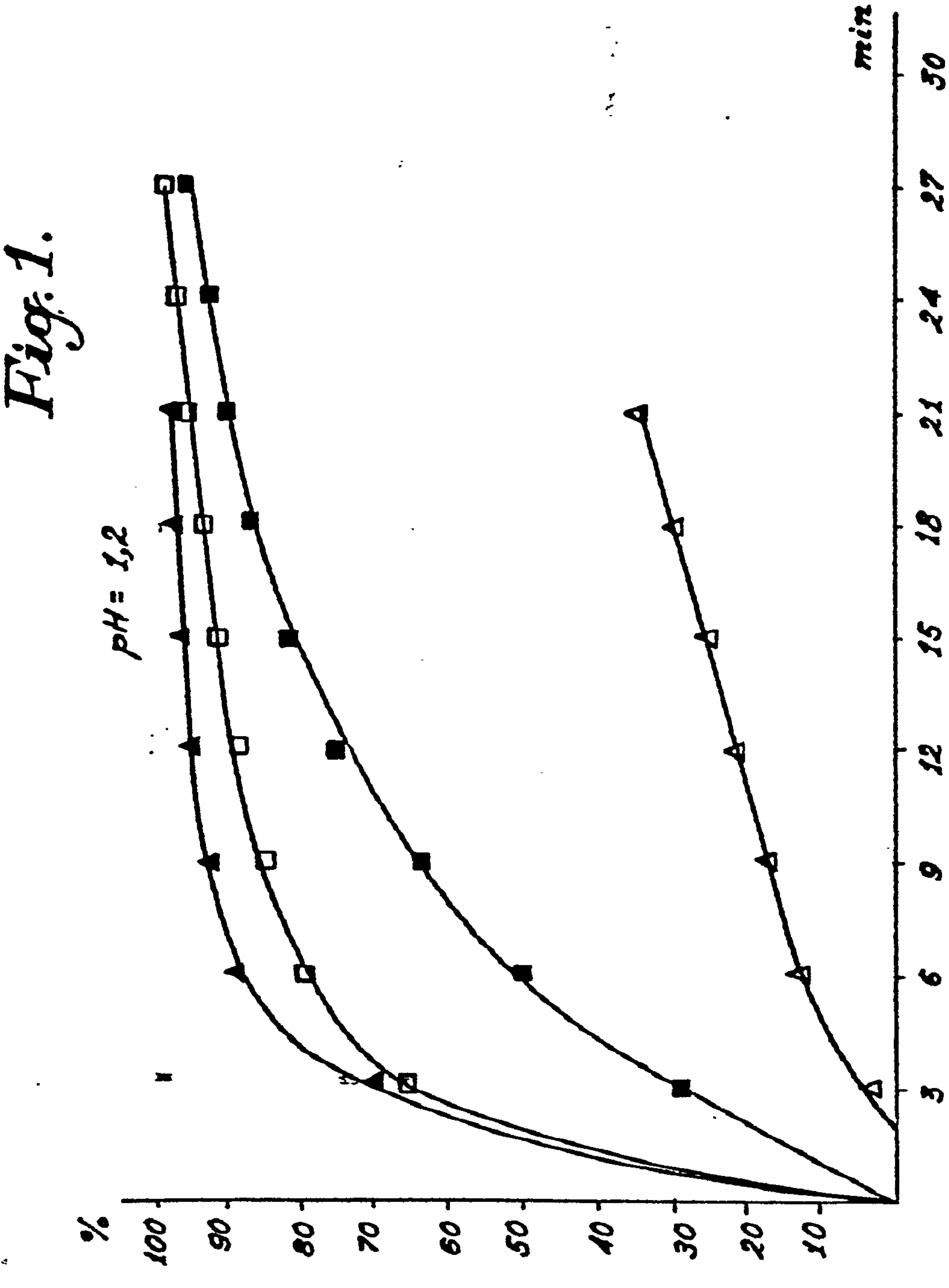

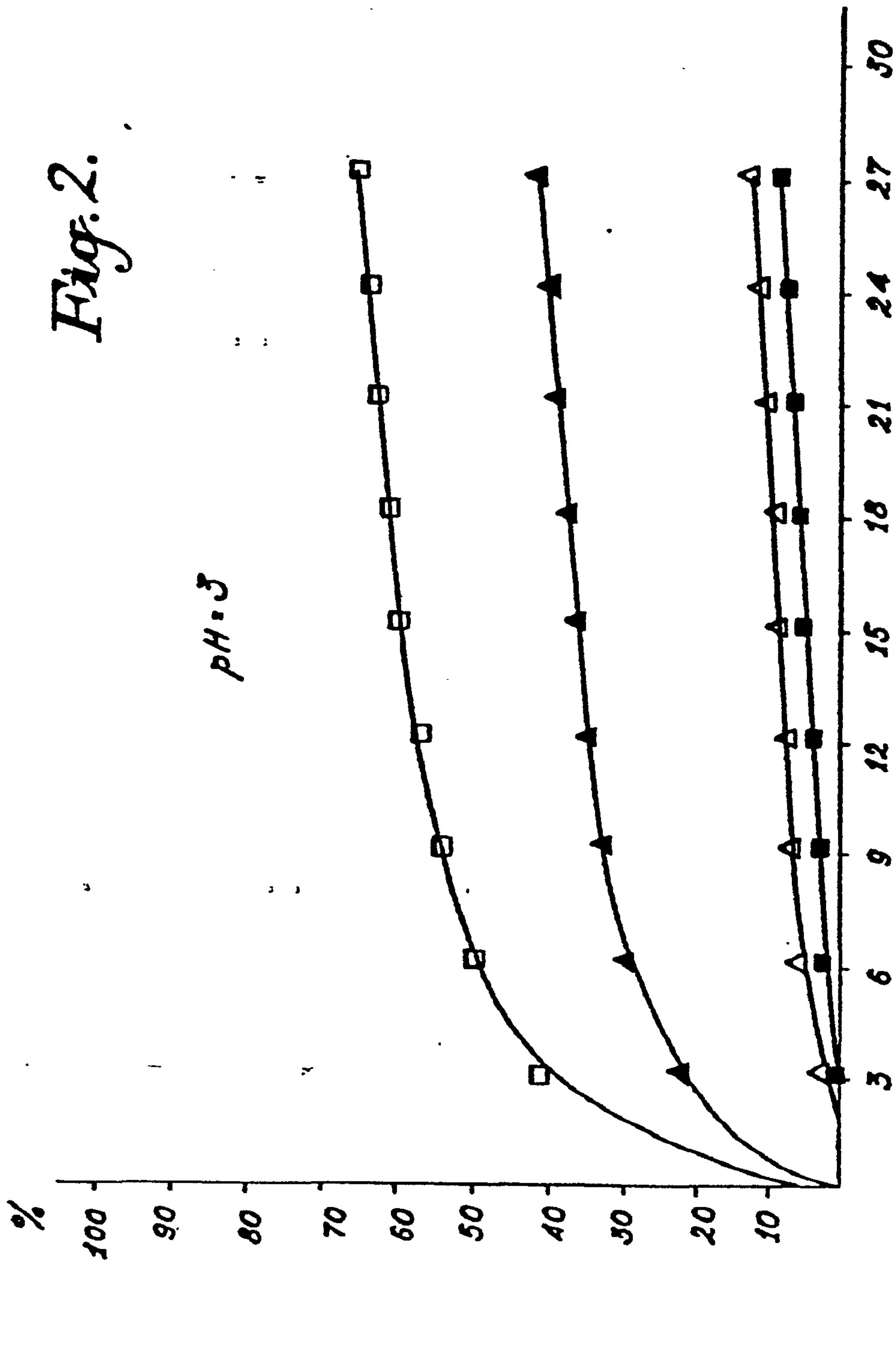


Fig. 2.

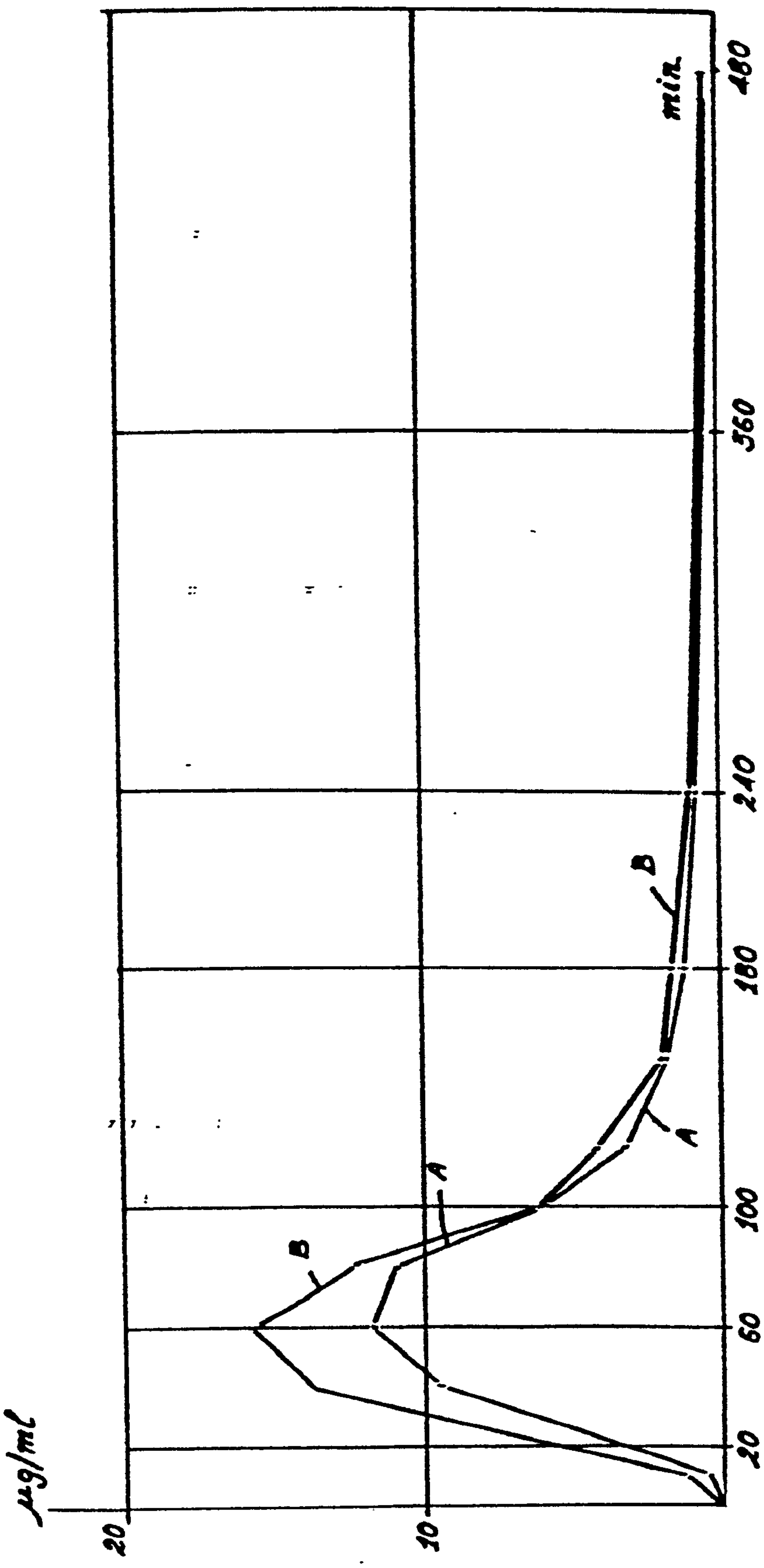


Fig. 3.

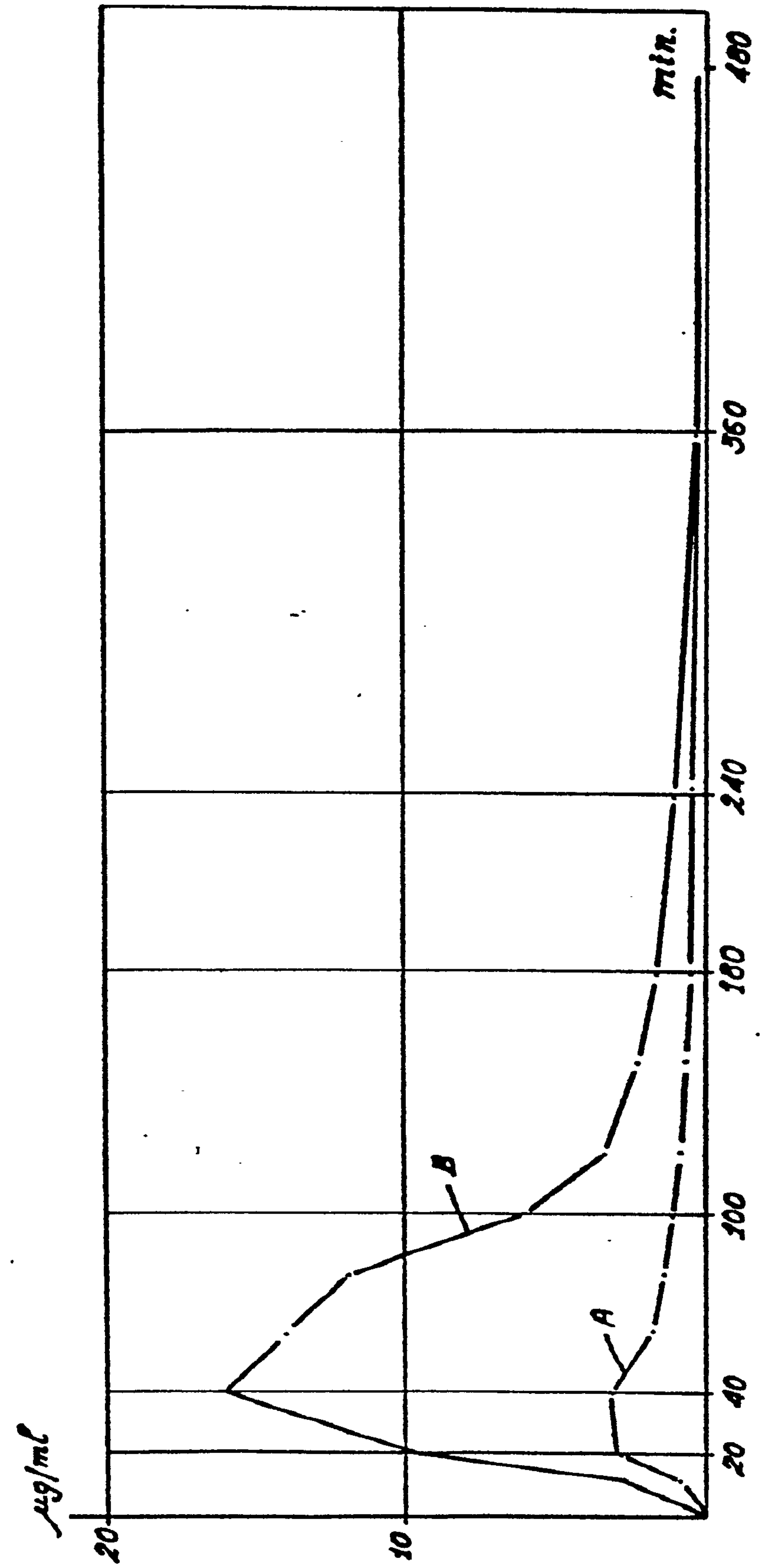


Fig.4.